(19) Europäisches Patentamt
European Patent Office
Office européen des brevets

(11) Veröffentlichungsnummer: **0 563 705 A2**

(12) # EUROPÄISCHE PATENTANMELDUNG

(21) Anmeldenummer: **93104552.0**

(22) Anmeldetag: **19.03.93**

(51) Int. Cl.5: **C07D 233/64**, C07D 405/06, C07D 409/06, C07D 401/06, A61K 31/415

(30) Priorität: **01.04.92 DE 4210787**

(43) Veröffentlichungstag der Anmeldung: **06.10.93 Patentblatt 93/40**

(84) Benannte Vertragsstaaten: **AT BE CH DE DK ES FR GB GR IE IT LI LU MC NL PT SE**

(71) Anmelder: **BAYER AG**

**D-51368 Leverkusen(DE)**

(72) Erfinder: **Krämer, Thomas, Dr.**
**In den Birken 92a**
**W-5600 Wuppertal 1(DE)**
Erfinder: **Dressel, Jürgen D., Ph.**
**Claudiusweg 9**
**W-5600 Wuppertal 1(DE)**
Erfinder: **Fey, Peter, Dr.**
**Am Eickhof 23**
**W-5600 Wuppertal 1(DE)**
Erfinder: **Hanko, Rudolf, Dr.**
**Schillerstrasse 23**
**W-4000 Düsseldorf 1(DE)**
Erfinder: **Hübsch, Walter, Dr.**
**Wildsteig 22**
**W-5600 Wuppertal 1(DE)**
Erfinder: **Müller, Ulrich, Dr.**
**Claudiusweg 5**
**W-5600 Wuppertal 1(DE)**
Erfinder: **Müller-Gliemann, Matthias, Dr.**
**Laibacher Strasse 10**
**W-5650 Solingen-Ohligs(DE)**
Erfinder: **Beuck, Martin, Dr.**
**Trills 7**
**W-4006 Erkrath 2(DE)**
Erfinder: **Kazda, Stanislav, Prof. Dr.**
**Gellertweg 4**
**W-5600 Wuppertal 1(DE)**
Erfinder: **Knorr, Andreas, Dr.**
**Trillser Graben 10**
**W-4006 Erkrath 2(DE)**
Erfinder: **Stasch, Johannes-Peter, Dr.**
**Schneewittchenweg 37**
**W-5600 Wuppertal 1(DE)**
Erfinder: **Wohlfeil, Stefan, Dr.**
**Tucherweg 25**
**W-4010 Hilden(DE)**

(54) **Cycloalkyl- und Heterocyclyl substituierte Imidazolylpropensäurederivate.**

(57) Cycloalkyl- und Heterocyclyl substituierte Imidazolylpropensäurederivate können durch Umsetzung von Imidazolylaldehyden mit entsprechen Carbonsäurederivaten und anschließende Dehydrierung hergestellt werden. Die Verbindungen können als Wirkstoffe in Arzneimitteln eingesetzt werden, insbesondere zur Behandlung von Hypertonie und Atherosklerose.

EP 0 563 705 A2

Die Erfindung betrifft Cycloalkyl- und Heterocyclyl substituierte Imidazolylpropensäurederivate, ein Verfahren zu ihrer Herstellung und ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Aus den Publikationen EP 403 159 A2 und EP 425 211 A1 sind Imidazolylderivate bekannt, die eine Angiotensin II-Rezeptor blockierende Wirkung besitzen.

Die vorliegende Erfindung betrifft Cycloalkyl- und Heterocyclyl substituierte Imidazolylpropensäurederivate der allgemeinen Formel (I)

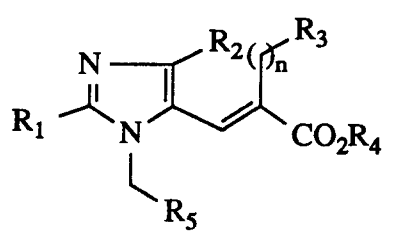

(I)

in welcher

$R^1$ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder für Cycloalkyl mit 3 bis 8 Kohlenstoffatomen steht,

$R^2$ für Wasserstoff, Halogen, Hydroxy, Nitro, Cyano, Trifluormethyl, Trifluormethoxy oder für Pentafluorethyl steht, oder für geradkettiges oder verzweigtes Alkyl mit bis zu 6 Kohlenstoffatomen steht, oder für Aryl mit 6 bis 10 Kohlenstoffatomen steht,

$R^3$ für Cycloalkyl mit 3 bis 8 Kohlenstoffatomen oder für Heterocyclyl mit 3 bis 8 Kohlenstoffatomen und mit bis zu 3 Heteroatomen aus der Reihe S, O oder -N-A steht,

worin

A Wasserstoff oder geradkettiges oder verzweigtes Alkyl mit bis zu 6 Kohlenstoffatomen bedeutet,

n für die Zahl 0, 1, 2, 3 oder 4 steht,

$R^4$ für Wasserstoff, geradkettiges oder verzweigtes Alkyl mit bis zu 8 Kohlenstoffatomen oder Phenyl steht,

$R^5$ für einen Rest der Formel

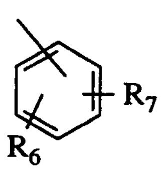 , 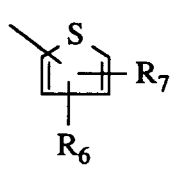 , 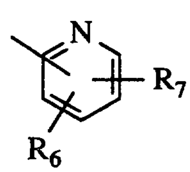 oder 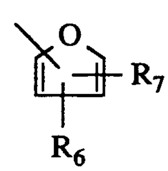

steht,

worin

$R^6$ Wasserstoff, Halogen, Cyano, Nitro, Trifluormethyl, Hydroxy, Trifluormethoxy, für geradkettiges oder verzweigtes Alkyl oder Alkoxy mit jeweils bis zu 6 Kohlenstoffatomen bedeutet,

$R^7$ Carboxy, geradkettiges oder verzweigtes Alkoxycarbonyl mit bis zu 6 Kohlenstoffatomen oder gegebenenfalls $C_1$-$C_4$-alkyliertes Tetrazolyl bedeutet,

und deren Salze.

Die erfindungsgemäßen Verbindungen können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Cycloalkyl- und Heterocycloalkyl substituierten Imidazolylpropensäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbederkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Heterocycloalkyl steht im allgemeinen für einen 5- bis 6-gliedrigen Ring mit bis zu 2 Heteroatomen aus der Reihe N, S, O oder einer NH-Gruppe bzw. N-$C_1$-$C_6$-Alkylgruppe, wie beispielsweise Dithiolanyl, Pyranyl oder Piperidyl. Bevorzugt sind Dithiolanyl oder Pyranyl.

Bevorzugt sind Verbindungen der allgemeinen Formel (I),

in welcher

$R^1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, oder für Cyclopropyl, Cyclopentyl oder Cyclohexyl steht,

$R^2$ für Wasserstoff, Fluor, Chlor, Brom, Iod, Trifluormethyl, Trifluormethoxy, Pentafluorethyl oder für geradkettiges oder verzweigtes Alkyl mit bis zu 6 Kohlenstoffatomen steht,

$R^3$ für Cyclopropyl, Cyclopentyl, Cyclohexyl, Cycloheptyl, Dithiolanyl oder Pyranyl steht,

n für die Zahl 0, 1, 2 oder 3 steht,

$R^4$ für Wasserstoff oder für geradkettiges oder verzweigtes Alkyl mit bis zu 6 Kohlenstoffatomen steht,

$R^5$ für einen Rest der Formel

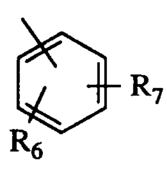 , 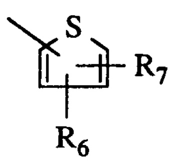 , 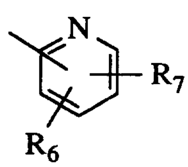 oder 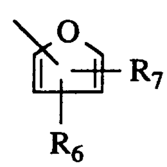

steht,

worin

$R^6$ Wasserstoff, Fluor, Chlor, Brom, Trifluormethyl, Trifluormethoxy oder geradkettiges oder verzweigtes Alkyl mit bis zu 4 Kohlenstoffatomen bedeutet,

$R^7$ Carboxy, geradkettiges oder verzweigtes Alkoxycarbonyl mit bis zu 3 Kohlenstoffatomen oder Tetrazolyl bedeutet

und deren Salze.

Besonders bevorzugt sind Verbindungen der allgemeinen Formel (I),

in welcher

$R^1$ für geradkettiges oder verzweigtes Alkyl oder Alkenyl mit jeweils bis zu 4 Kohlenstoffatomen oder Cyclopropyl steht,

$R^2$ für Wasserstoff, Fluor, Chlor, Brom, Iod, Trifluormethyl, Pentafluorethyl, Trifluormethoxy oder für geradkettiges oder verzweigtes Alkyl mit bis zu 4 Kohlenstoffatomen steht,

$R^3$ für Cyclopropyl, Cyclopentyl oder Cyclohexyl steht,

n für die Zahl 0, 1 oder 2 steht,

$R^4$ für Wasserstoff oder für geradkettiges oder verzweigtes Alkyl mit bis zu 4 Kohlenstoffatomen steht,

$R^5$ für einen Rest der Formel

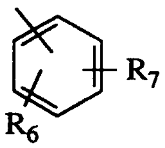
,
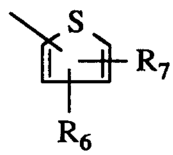
,
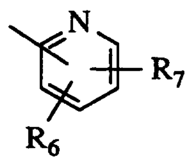
oder
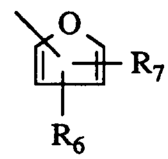

steht,
worin

$R^6$ Wasserstoff, Fluor, Chlor oder Brom bedeutet,

$R^7$ Carboxy, geradkettiges oder verzweigtes Alkoxycarbonyl mit bis zu 3 Kohlenstoffatomen oder Tetrazolyl bedeutet

und deren Salze.

Außerdem wurde ein Verfahren zur Herstellung der erfindungsgemäßen Verbindungen der allgemeinen Formel (I) gefunden, dadurch gekennzeichnet, daß man
Aldehyde der allgemeinen Formel (II)

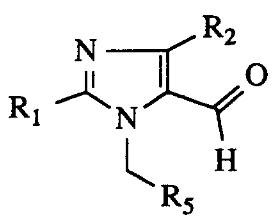
(II)

in welcher
$R^1$, $R^2$ und $R^5$ die oben angegebene Bedeutung haben,
zunächst durch Umsetzung mit Verbindungen der allgemeinen Formel (III)

$$R^3\text{-}(CH_2)_n\text{-}CO_2\text{-}T \qquad (III)$$

in welcher
$R^3$ und n die oben angegebene Bedeutung haben,
und

T für geradkettiges oder verzweigtes Alkyl mit bis zu 8 Kohlenstoffatomen steht oder Phenyl bedeutet,

in inerten Lösemitteln, in Anwesenheit einer Base in die Verbindungen der allgemeinen Formel (IV)

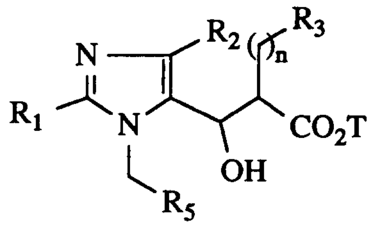

(IV)

in welcher
$R^1$, $R^2$, $R^3$, n und T die oben angegebene Bedeutung haben,
überführt,
anschließend die freie Hydroxyfunktion durch Einführung einer Schutzgruppe blockiert und in einem letzten Schritt eine Eliminierung in inerten Lösemitteln in Anwesenheit einer Base durchführt,
und im Fall der Säuren ($R^4/R^7$ = H) die Ester verseift,
und gegebenenfalls die Substituenten $R^1$ und $R^2$ nach üblichen Methoden, beispielsweise durch Hydrierung oder Alkylierung variiert,
und im Fall, daß $R^7$ für alkyliertes Tetrazolyl steht, die -NH-Funktion alkyliert.

Das erfindungsgemäße Verfahren kann durch folgendes Formelschema beispielhaft erläutert werden:

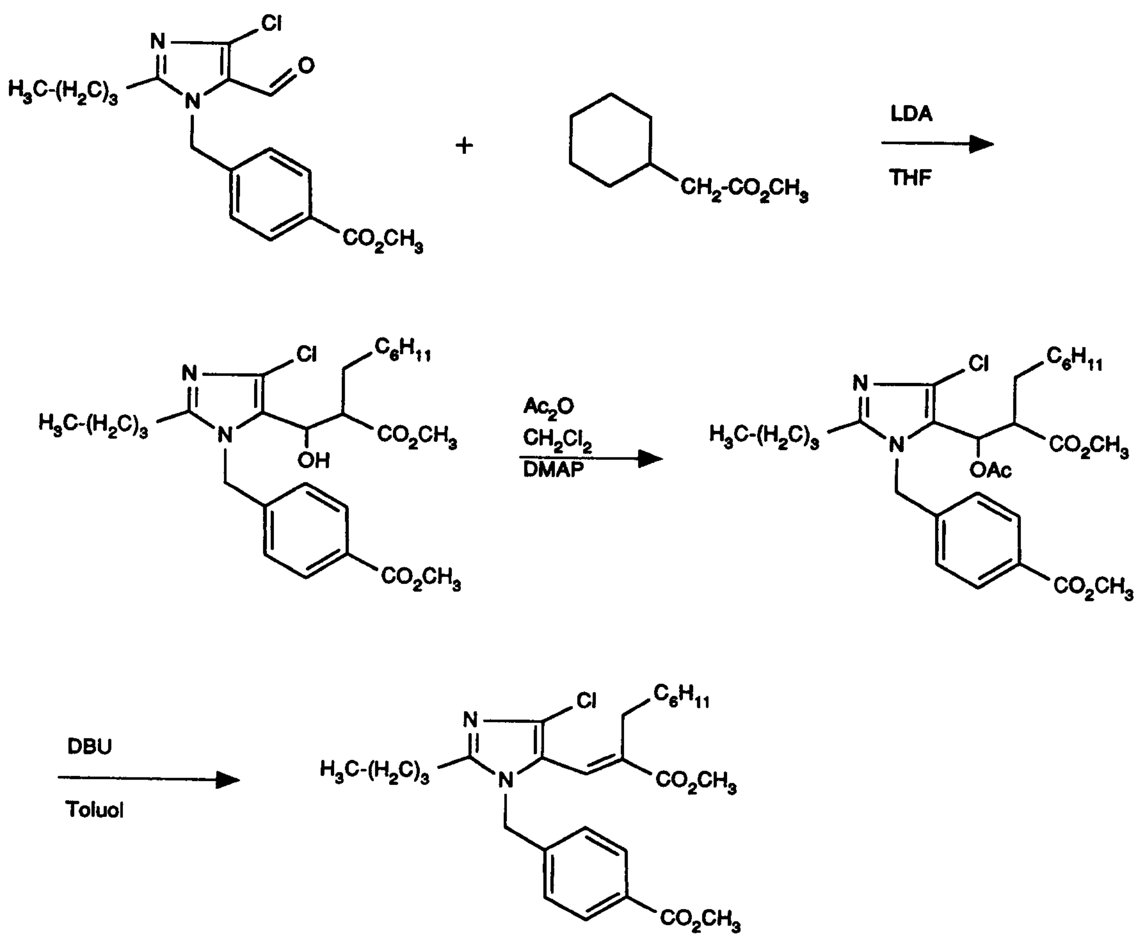


Hydroxyschutzgruppe im Rahmen der oben angegebenen Definition steht im allgemeinen für eine Schutzgruppe aus der Reihe: Benzyloxycarbonyl, Methansulfonyl, Toluolsulfonyl, 2-Nitrobenzyl, 4-Nitrobenzyl, 2-Nitrobenzyloxycarbonyl, 4-Nitrobenzyloxycarbonyl, tert.Butoxycarbonyl, Allyloxycarbonyl, 4-Methoxycarbonyl, Acetyl, Trichloracetyl, 2,2,2-Trichlorethoxycarbonyl, 2,4-Dimethoxybenzyloxycarbonyl, 2-(Methylthiomethoxy)ethoxycarbonyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Methansulfonyl und Toluolsulfonyl.

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind für die verschiedenen Schritte Tetrahydrofuran, Methylenchlorid und Toluol.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind Lithiumdiisopropylamid und DBU.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -100°C bis +100°C, bevorzugt bei -78°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Einführung der Schutzgruppe erfolgt im allgemeinen in einem der oben aufgeführten Lösemitteln und einer Base,vorzugsweise in Methylenchlorid mit Dimethylaminopyridin.

Die Blockierung erfolgt im allgemeinen in einem Temperturbereich von 0°C bis +60°C, vorzugsweise bei Raumtemperatur und bei Normaldruck.

Die Eliminierung wird im allgemeinen in einem der oben aufgeführten Lösemitteln, vorzugsweise in Toluol und in Anwesenheit einer der aufgeführten Basen, vorzugsweise DBU durchgeführt.

Die Eliminierung erfolgt im allgemeinen in einem Temperaturbereich von +30°C bis +130°C, vorzugsweise bei +50°C bis +100°C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die

basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Alkylierung erfolgt im allgemeinen mit Alkylierungsmitteln wie beispielsweise ($C_1$-$C_6$)-Alkylhalogeniden, Sulfonsäureestern oder substituierten oder unsubstituierten ($C_1$-$C_6$)-Dialkyl- oder ($C_1$-$C_6$)-Diarylsulfonate, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Die Hydrierung verläuft im allgemeinen nach üblichen Methoden, in einem der oben aufgeführten Lösemitteln, vorzugsweise in Methanol, mit Wasserstoff und Pd/C in Anwesenheit von Natriumacetat.

Im allgemeinen wird die Hydrierung bei erhöhtem Druck (ca 2 bis 3 bar) durchgeführt. Es ist aber auch möglich, bei Unterdruck oder bei Normaldruck zu arbeiten.

Die Hydrierung wird im allgemeinen in einem Temperaturbereich von 0 °C bis +40 °C, bevorzugt bei Raumtemperatur durchgeführt.

Die Verbindungen der allgemeinen Formel (II) sind teilweise bekannt ($R^5$ = Phenyl, vgl. hierzu beispielsweise EP 425 211) oder können hergestellt werden, indem man
Verbindungen der allgemeinen Formel (V)

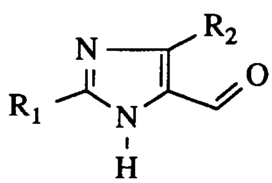

(V)

in welcher
$R^1$ und $R^2$ die oben angegebene Bedeutung haben,
mit Verbindungen der allgemeinen Formel (VI)

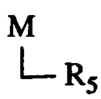

(VI)

in welcher
$R^5$ die oben angegegebene Bedeutung hat
und
M für Halogen, vorzugsweise für Brom steht,
in einem der oben aufgeführten Lösemittel, vorzugsweise Dimethylformamid in Anwesenheit einer der oben aufgeführten Basen, vorzugsweise Kaliumcarbonat oder Kalium-tert.butylat umsetzt.

Die Verbindungen der allgemeinen Formeln (V) und (VI) sind bekannt.

Die Verbindungen der allgemeinen Formel (III) sind bekannt oder können nach üblicher Methode durch Veresterung der käuflichen Säuren hergestellt werden [vgl. z.B. MSD Book 2, 1593 D].

Die Verbindungen der allgemeinen Formel (IV) sind als konkrete Stoffvertreter neu und können beispielsweise nach dem oben aufgeführten Verfahren hergestellt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen Cycloalkyl- und Hetercyclyl-substituierten Imidazolylpropensäurederivate zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die

Proliferation von glatten Muskelzellen.

Sie können deshalb in Arzneimittel zur Behandlung der arterieller Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogenbegaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

Agonisten und ihre Standardkonzentrationen Applikationsvolumen pro Einzelgabe (= 100 µl):

| | | |
|---|---|---|
| KCl | 22,7;32,7;42,7;52,7 | mmol/l |
| 1-Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. (= submaximalen) Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark und Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3$H-Angiotensin II (3-5 nM), Test-Pufferlösung (50 mM Tris, pH 7,2, 5 mM $MgCl_2$, 0,25% BSA) sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$.-Werte; $IC_{50}$-Werte: Konzentration bei der die zu untersuchende Substanz eine 50%ige Hemmung der totalen Bindung des Radioliganden bewirkt).

Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt.

Zur Bestimmung der $IC_{50}$-Werte wird die Wirkstoffkonzentration errechnet, die bei sequentieller Verdünnung des Wirkstoffes halbmaximale Hemmung der durch 10 % FCS hervorgerufene Thymidininkorporation bewirkt.

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

Ausgangsverbindungen

Beispiel I

4-Brommethyl-benzoesäuremethylester

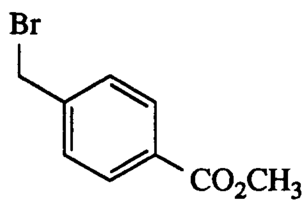

45 g (0,3 mol) p-Tolylsäuremethylester und 53,4 g (0,3 mol) N-Bromsuccinimid werden zusammen mit 1,97 g Azobisisobutyronitril in 375 ml Tetrachlorkohlenstoff 1 h zum Sieden erhitzt, anschließend auf 0°C abgekühlt und vom Succinimid abfiltriert. Der Rückstand wird über Kieselgel 60 mit Essigester / Petrolether (1:10) chromatographiert.
Ausbeute: 55,7 g (81% der Theorie)
RF = 0,58 (Essigester / Petrolether = 1:10)

Beispiel II

4-Brommethyl-3-chlor-benzoesäuremethylester

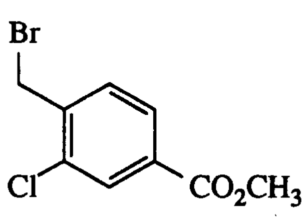

In Analogie zur Vorschrift des Beispiels I wird die Titelverbindung aus 9,0 g (48,8 mmol) 3-Chlor-p-tolylsäuremethylester hergestellt.
Ausbeute: 7,3 g (57% der Theorie)
$R_F$ = 0,62 (Essigester / Petrolether = 1:5)

Beispiel III

5-Brommethylthiophen-2-carbonsäuremethylester

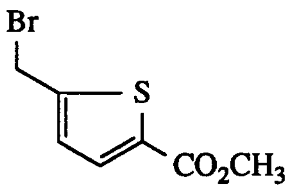

In Analogie zur Vorschrift des Beispiels I wird die Titelverbindung aus 20 g (0,13 mol) 5-Methylthiophen-2-carbonsäuremethylester hergestellt.
Ausbeute: 22 g (73% der Theorie)
$R_F$ = 0,43 (Dichlormethan / Methanol = 10:1)

Beispiel IV

4-[(2-n-Butyl-4-chlor-5-formyl-imidazol-1-yl)methyl]-benzoesäuremethylester

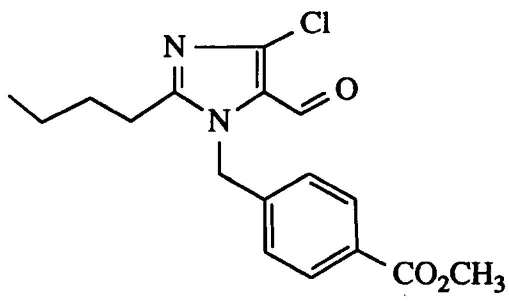

Unter Schutzgas werden 20 g (0,107 mol) 2-n-Butyl-4-chlor-5-formylimidazol (Herstellung nach EP 324 377) in DMF mit 13 g (0,107 mol) Kalium-tert.butylat 30 min bei 25 °C gerührt, anschließend 36 g (0,157 mol) der Verbindung aus Beispiel I in DMF zugetropft und 20 h bei 25 °C gerührt. Nach Einengen wird das Rohprodukt an Kieselgel 60 mit Essigester / Petrolether (1:2) chromatographiert.
Ausbeute: 25,5 g (69% der Theorie)
$R_F$ = 0,7 (Essigester / Petrolether = 1:2)

Beispiel V

4-[(2-n-Butyl-4-chlor-5-formyl-imidazol-1-yl)methyl]-3-chlor-benzoesäuremethylester

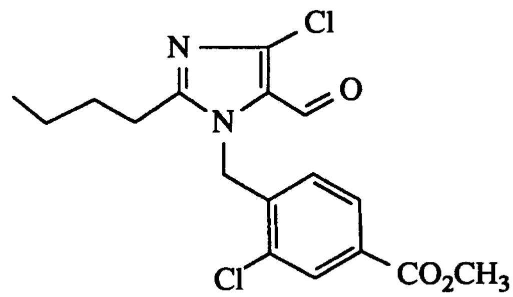

In Analogie zur Vorschrift des Beispiels IV wird die Titelverbindung aus 3,16 g (12 mmol) der Verbindung aus Beispiel II hergestellt.
Ausbeute: 2,3 g (78% der Theorie)
$R_F$ = 0,38 (Essigester / Petrolether = 1:5)

Beispiel VI

5-[(2-n-Butyl-4-chlor-5-formyl-imidazol-1-yl)methyl]-thiophen-2-carbonsäuremethylester

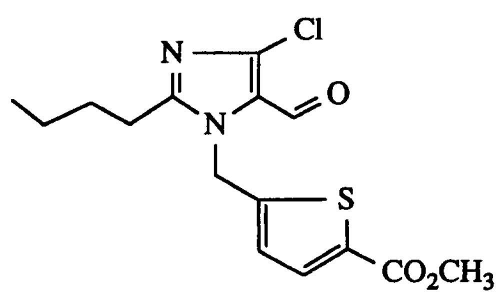

14,4 g (77 mmol) 2-n-Butyl-4-chlor-5-formylimidazol, 20 g (85 mmol) der Verbindung aus Beispiel III und 21,3 g (154 mmol) Kaliumcarbonat werden in 300 ml DMF 20 h bei 25 °C gerührt. Nach Einengen wird der Rückstand in 300 ml Dichlormethan und 250 ml Wasser aufgenommen, die organische Phase abgetrennt, über Natriumsulfat getrocknet und eingeengt. Das so erhaltene Produkt wird an

Kieselgel 60 mit Essigester / Petrolether (1:3) chromatographiert.

Ausbeute: 14,9 g (57% der Theorie) $R_F$ = 0,54 (Essigester / Petrolether = 1:3)

Beispiel VII

4-[(2-n-Butyl-5-formyl-imidazol-1-yl)methyl]-benzoesäuremethylester

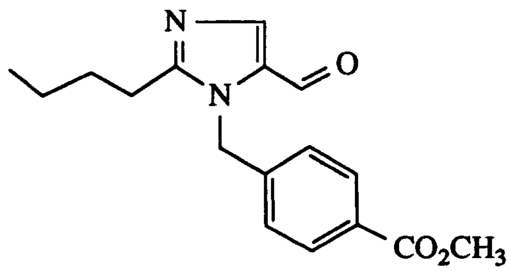

20 g (63 mmol) der Verbindung aus Beispiel IV in 300 ml Methanol werden bei 25 ° C in Gegenwart von 2 g Palladium auf Aktivohle (5%ig) und 8,6 g (63 mmol) Natriumacetat-Trihydrat 3 h bei ca. 3 bar Wasserstoffdruck hydriert. Die Lösung wird dann vom Katalysator abfiltriert und konzentriert. Danach wird der Rückstand

über Kieselgel vom Essigester / Petrolether (3:1) chromatographiert

Ausbeute: 13,4 g (75% der Theorie) $R_F$ = 0,48 (Essigester / Petrolether = 3:1)

Beispiel VIII

4-[(2-n-Butyl-5-formyl-imidazol-1-yl)methyl]-3-chlor-benzoesäuremethylester

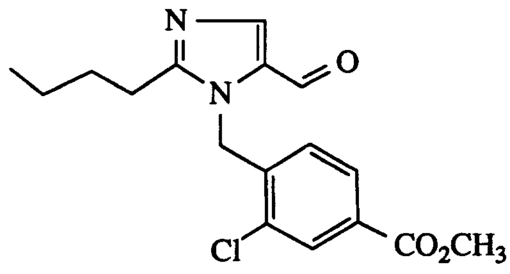

In Analogie zur Vorschrift des Beispiels VII wird die Titelverbindung aus 3,1 g (8,4 mmol) der Verbindung aus Beispiel II hergestellt.
Ausbeute: 2 g (71% der Theorie)
$R_F$ = 0,54 (Essigester / Petrolether = 5:1)

Beispiel IX:

5-[(2-n-Butyl-4-chlor-5-formylimidazol-1-yl)methyl]-furan-2-carbonsäureethylester

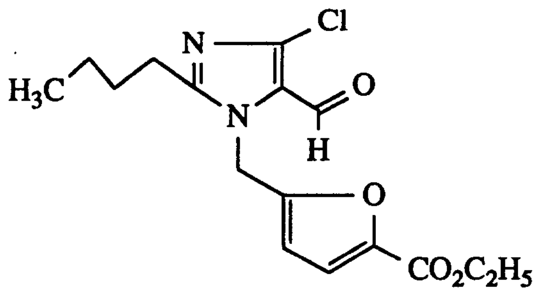

In Analogie zur Vorschrift des Beispiels VI wurde die Titelverbindung aus 18,7 g (0,1 mol) 2-n-Butyl-4-chlor-5-formylimidazol und 24,1 g (0,103 mol) 5-Brommethylfuran-2-carbonsäureethylester hergestellt.
Ausbeute: 22,8 g (65% der Theorie)
$R_F$ = 0,38 (Essigester / Petrolether = 1:2)

Beispiel X:

5-[(2-n-Butyl-5-formyl-imidazol-1-yl)methyl]-furan-2-carbonsäureethylester

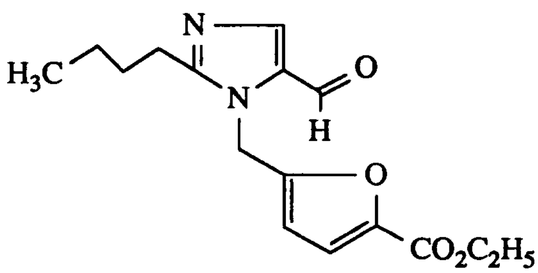

In Analogie zur Vorschrift des Beispiels VII wurde die Titelverbindung aus 14,51 g (42 mmol) der Verbindung aus Beispiel IX hergestellt.
Ausbeute: 7,31 g (57% der Theorie)
$R_F$ = 0,39 (Essigester / Petrolether = 1:1)

Beispiel XI:

5-[(2-n-Butyl-5-formyl-imidazol-1-yl)methyl]-thiophen-2-carbonsäureethylester

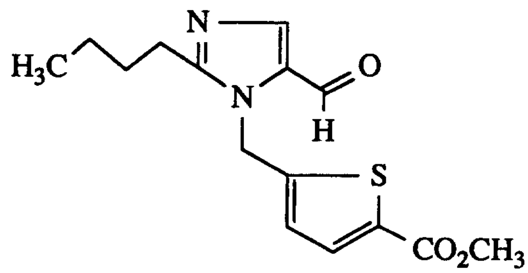

In Analogie zur Vorschrift des Beispiels VII wurde die Titelverbindung aus 6,5 g (19,1 mmol) der Verbindung aus Beispiel VI hergestellt.
Ausbeute: 4 g (68% der Theorie)
$R_F$ = 0,21 (Essigester / Petrolether = 1:1)

Beispiel XII:

3-[2-n-Butyl-1-{(2-ethoxycarbonylfuran-5-yl)methyl}-1H-imidazol-5-yl]-2-cyclopentylmethyl-3-hydroxy-propionsäure-methylester

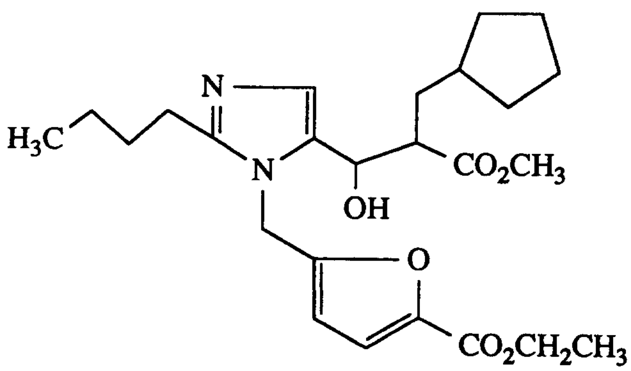

Zu einer Lösung von 0,89 g (8,75 mmol) N,N-Diisopropylamin in 10 ml THF werden unter Schutzgas bei -78°C 5,16 ml (8,25 mmol) einer 1,6 N Lösung von n-Butyllithium in n-Hexan injiziert Anschließend wird die Reaktionslösung kurz auf O°C erwärmt, erneut auf -78°C gekühlt und 1,17 g (7,5 mmol) 3-Cyclopentylpropionsäuremetylester in 5 ml THF zugegeben. Es wird 30 min bei -78°C gerührt, 1,52 g (5 mmol) der Verbindung aus Beispiel X in 15 ml THF zugegeben und 45 min bei -78°C nachgerührt. Danach wird langsam auf 25°C erwärmt, 20 ml ges. Ammoniumchlorid-Lösung zugegeben und dreimal mit je 50 ml Essigester extrahiert. Die organische Phase wird über Natriumsulfat getrocknet, eingeengt und der Rückstand an Kieselgel mit Essigester/Petrolether (2:1) gereinigt.
Ausbeute: 1,1 g (48% der Theorie)
$R_F$ = 0,09 (Essigester / Petrolether = 2:1, Diastereomerengemisch)

Beispiel XIII:

3-[2-n-Butyl-1-{(4-methoxycarbonylphenyl)methyl}-1H-imidazol-5-yl]-2-cyclopentyl-methyl-3-hydroxy-propionsäure-methylester

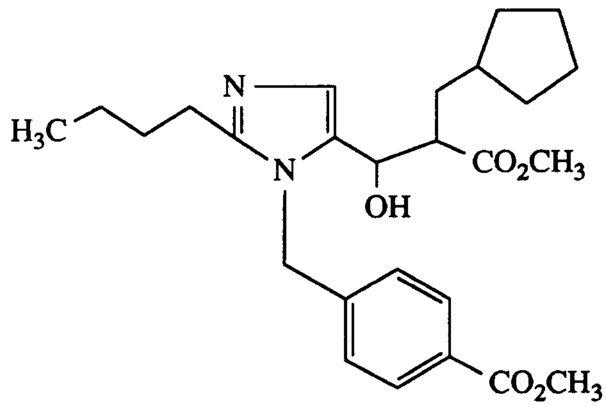

In Analogie zur Vorschrift des Beispiels XII wurde die Titelverbindung aus 885 mg (8,75 mmol) der Verbindung aus Beispiel VII hergestellt und ohne Reinigung weiter umgesetzt.
$R_F$ = 0,17 (Essigester / Petrolether = 4:1, Diastereomerengemisch)

Beispiel XIV:

3-[2-n-Butyl-1-{(2-methoxycarbonylthiophen-5-yl)methyl}-1H-imidazol-5-yl]-2-cyclopentyl-methyl-3-hydroxy-propionsäure-methylester

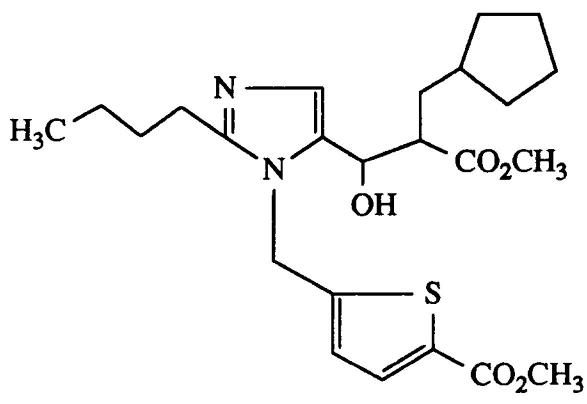

In Analogie zur Vorschrift des Beispiels XII wurde die Titelverbindung aus 1,53 g (5 mmol) der Verbindung aus Beispiel XI hergestellt.
Ausbeute: 1,25 g (54 % der Theorie)
$R_F$ = 0,12 (Essigester / Petrolether = 2:1, Diastereomerengemisch)

Beispiel XV:

3-Acetoxy-3-[2-n-butyl-1-{(2-ethoxycarbonylfuran-5-yl)methyl}-1H-imidazol-5-yl]-2-cyclopentylmethyl-propionsäure-methylester

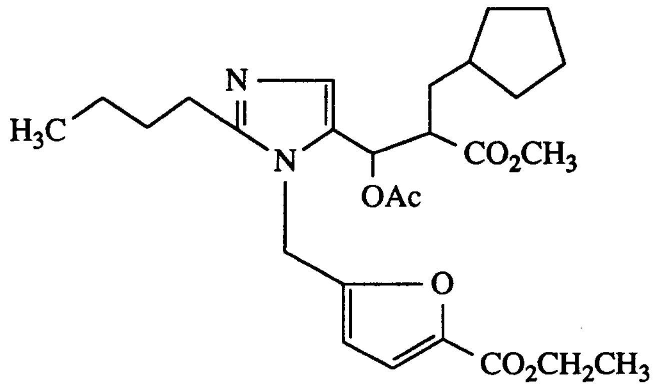

2,45 g (5,36 mmol) der Verbindung aus Beispiel XII werden in 50 ml Dichlormethan gelöst, mit 264 mg (2,16 mmol) N,N'-Dimethylaminopyridin (DMAP) und 0,76 ml (8,04 mmol) Acetanhydrid versetzt und 16 h bei 25 °C gerührt. Man verdünnt mit Ether, wäscht mit Wasser (1 x 40 ml) und ges. Natriumhydrogencarbonat-Lösung, trocknet die organische Phase über Natriumsulfat und engt ein. Das so erhaltene Rohprodukt wird an Kieselgel mit Essigester/Petrolether (3:2) chromatographiert.
Ausbeute: 1,93 g (72% der Theorie)
$R_F$ = 0,65 (Essigester / Petrolether = 3:2)

Beispiel XVI:

3-Acetoxy-3-[2-n-butyl-1-{(4-methoxycarbonylphenyl)methyl}-1H-imidazol-5-yl]-2-cyclopentylmethyl-propionsäure-methylester

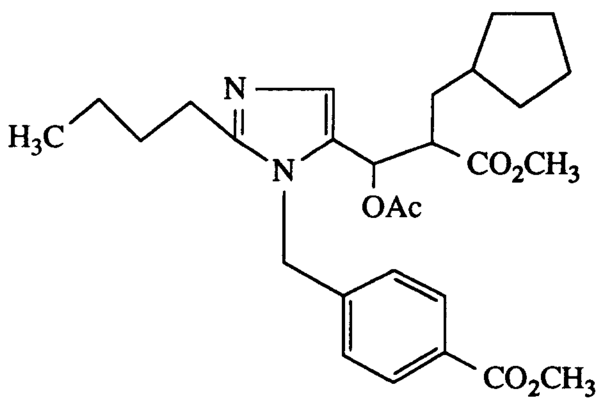

In Analogie zur Vorschrift des Beispiels XV wurde die Titelverbindung aus dem Rohprodukt des Beispiels XIII hergestellt.
Ausbeute: 1,7 g (66% der Theorie)
$R_F$ = 0,63 (Essigester / Petrolether = 4:1, Diastereomerengemisch)

Beispiel XVII:

3-Acetoxy-3-[2-n-butyl-1-{(2-methoxycarbonylthiophen-5-yl)methyl}-1H-imidazol-5-yl]-2-cyclopentylmethyl-propionsäure-methylester

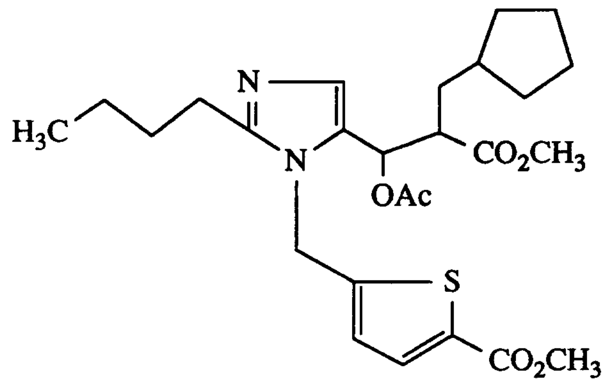

In Analogie zur Vorschrift des Beispiels XV wurde die Titelverbindung aus 1,2 g (2,6 mmol) der Verbindung aus Beispiel XIV hergestellt.
Ausbeute: 1,23 g (94% der Theorie)
$R_F$ = 0,67 (Essigester / Petrolether = 2:1, Diastereomerengemisch)

Herstellungsbeispiele

Beispiel 1

3-[2-n-Butyl-1-{(2-ethoxycarbonylfuran-5-yl)methyl}-1H-imidazol-5-yl]-2-cyclopentylmethyl-2-propensäure-methylester

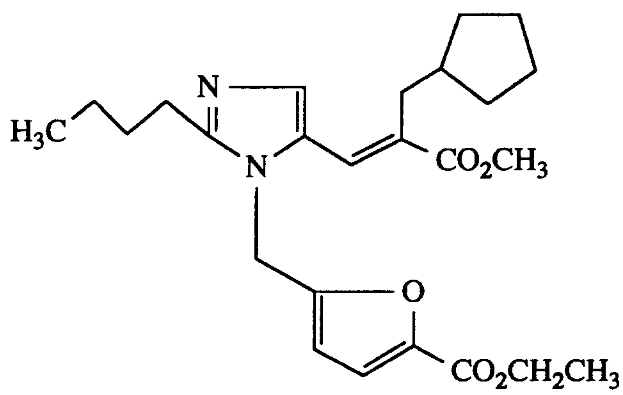

1,9 g (3,78 mmol) der Verbindung aus Beispiel XV werden in 60 ml Toluol gelöst, 1,13 ml (7,56 mmol) 1,8-Diazabicyclo[5.4.0]undec-7-en (DBU) zugegeben und 6 h bei 80°C gerührt. Danach werden erneut 1,13 ml (7,56 mmol) DBU zugegeben und der Kolbeninhalt 16 h bei 80°C gerührt. Nach Abkühlen wird mit ges. Natriumchlorid-Lösung (1 x 30 ml) gewaschen, die organische Phase über Natriumsulfat getrocknet, filtriert und eingeengt. Der Rückstand wird an Kieselgel mit Essigester/Petrolether (1:2) chromatographiert.
Ausbeute: 1,16g (69% der Theorie)
$R_F$ = 0,68 (Essigester / Petrolether = 1:1)

Beispiel 2:

3-[2-n-Butyl-1-{(4-methoxycarbonylphenyl)methyl}-1H-imidazol-5-yl]-2-cyclopentylmethyl-2-propensäure-methylester

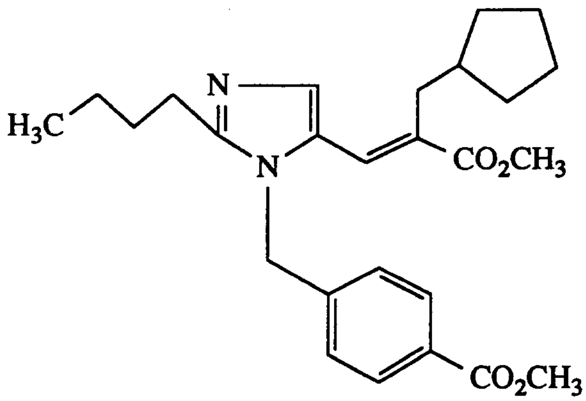

In Analogie zur Vorschrift des Beispiels 1 wurde die Titelverbindung aus 1,6 g (3,2 mmol) der Verbindung aus Beispiel XVI hergestellt.
Ausbeute: 0,61 g (43% der Theorie)
$R_F$ = 0,63 (Essigester / Petrolether = 2:1)

Beispiel 3:

3-[2-n-Butyl-1-{(4-methoxycarbonylthiophen-5-yl)methyl}-1H-imidazol-5-yl]-2-cyclopentylmethyl-2-propensäure-methylester

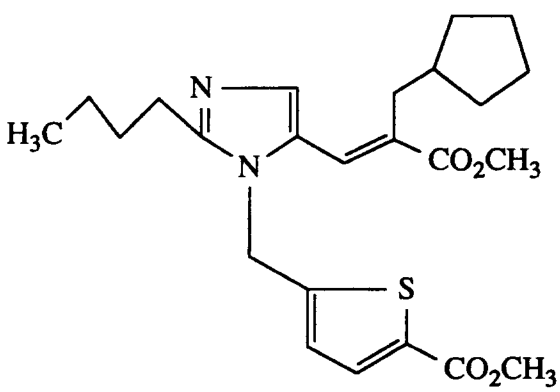

In Analogie zur Vorschrift des Beispiels 1 wurde die Titelverbindung aus 1,2 g (2,38 mmol) der Verbindung aus Beispiel XVII hergestellt.
Ausbeute: 0,5 g (47% der Theorie)
$R_F$ = 0,55 (Essigester / Petrolether = 1:1)

Beispiel 4:

3-[2-n-Butyl-1-{(4-carboxyphenyl)methyl}-1H-imidazol-5-yl]-2-cyclopentylmethyl-2-propensäure

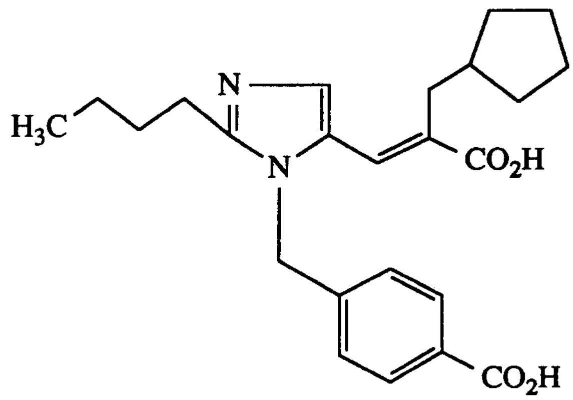

Zu einer Lösung von 1,26 g (2,88 mmol) der Verbindung aus Beispiel 2 in 30 ml Methanol wird eine Lösung von 1 g Natriumhydroxid in 20 ml Methanol/Wasser (1:1) gegeben. Man rührt 16 h bei 50 °C, säuert anschließend mit verd. Salzsäure an, gießt auf 300 ml Wasser und extrahiert dreimal mit je 75 ml Dichlormethan und dreimal mit je 40 ml Essigester. Die organischen Phasen werden über Natriumsulfat getrocknet, filtriert und vom Lösemittel befreit. Das Rohprodukt wird an Kieselgel (Toluol/Essigester/Eisessig 10:30:0,2→Toluol/Methanol/Eisessig 35:5:0,2) gereinigt. Man erhält 342 mg (29 % der Theorie).
$R_F$ = 0,25 (Toluol/Methanol/Eisessig = 35:5:1)

Beispiel 5:

3-[2-n-Butyl-1-{(2-carboxythiophen-5-yl)methyl}-1H-imidazol-5-yl]-2-cyclopentylmethyl-2-propensäure

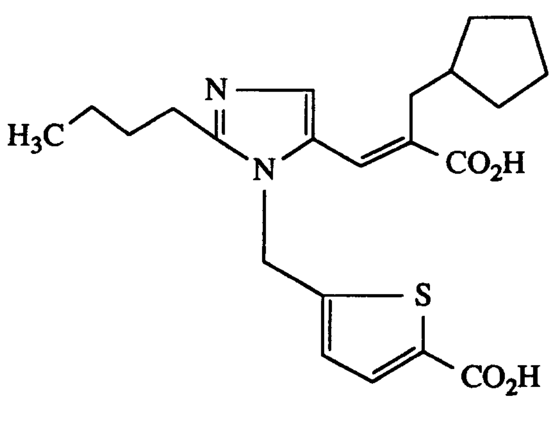

In Analogie zur Vorschrift des Beispiels 4 wurde die Titelverbindung aus 500 mg (1,13 mmol) der Verbindung aus Beispiel 3 hergestellt.
Ausbeute: 101 mg (21% der Theorie)
$R_F$ = 0,13 (Toluol/Essigester/Eisessig = 10:30:0,2)

Beispiel 6:

3-[2-n-Butyl-1-{(2-carboxyfuran-5-yl)methyl}-1H-imidazol-5-yl]-2-cyclopentylmethyl-2-propensäure

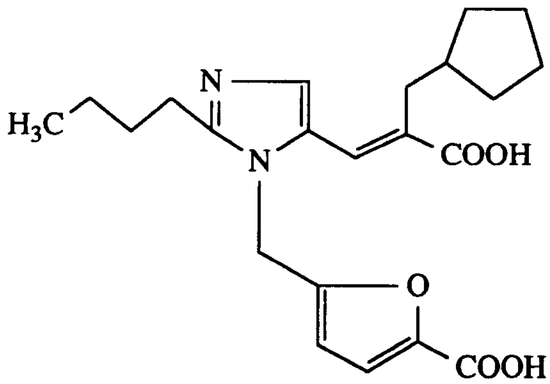

In Analogie zur Vorschrift des Beispiels 4 wurde die Titelverbindung aus 1,11 g (1,13 mmol) der Verbindung aus Beispiel 1 hergestellt.
Ausbeute: 380 mg (39% der Theorie)
$R_F$ = 0,07 (Toluol/Essigester/Eisessig = 10:30:1)

Beispiel 7:

3-[2-n-Butyl-1-{(4-carboxyphenyl)methyl}-1H-imidazol-5-yl]-2-cyclopentylmethyl-2-propensäure, di-Na-Salz

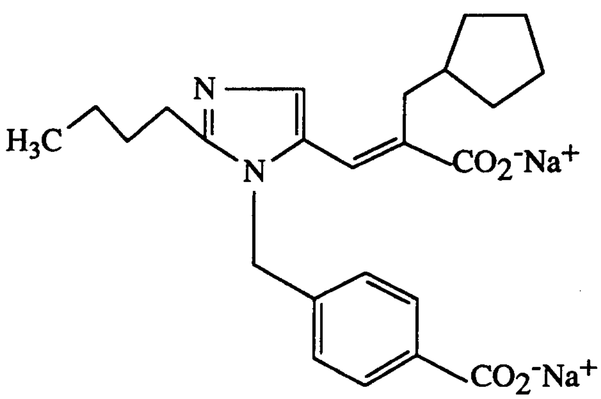

340 mg (0,83 mmol) der Verbindung aus Beispiel 4 werden in Methanol/Wasser gelöst, mit 1,66 ml einer 1 N Natronlauge versetzt und anschließend vom Lösemittel befreit. Man erhält 376 mg (100 % der Theorie).

Beispiel 8:

3-[2-n-Butyl-1-{(2-carboxythiophen-5-yl)methyl}-1H-imidazol-5-yl]-2-cyclopentylmethyl-2-propensäure, di-Na-Salz

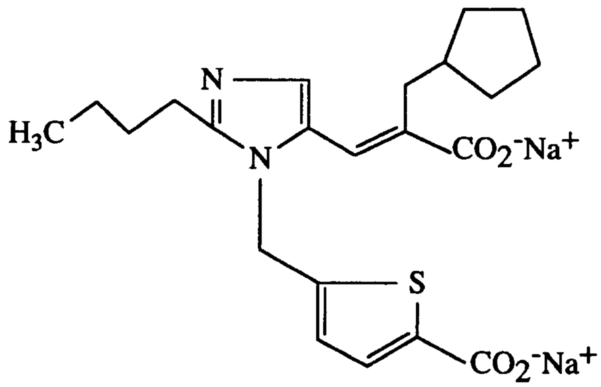

In Analogie zur Vorschrift des Beispiels 7 wurde die Titelverbindung aus 330 mg (0,79 mmol) der Verbindung aus Beispiel 5 hergestellt

Beispiel 9:

3-[2-n-Butyl-1-{(2-carboxyfuran-5-yl)methyl}-1H-imidazol-5-yl]-2-cyclopentylmethyl-2-propensäure, di-Na-Salz

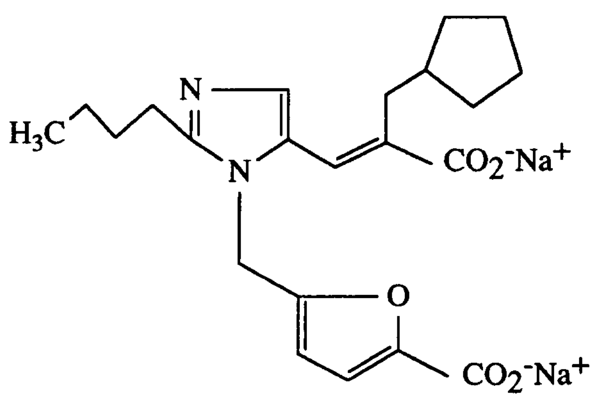

In Analogie zur Vorschrift des Beispiels 7 wurde die Titelverbindung aus 376 mg (0,94 mmol) der Verbindung aus Beispiel 6 hergestellt.

**Patentansprüche**

1. Cycloalkyl- und Heterocyclyl substituierte Imidazolylpropensäurederivate der allgemeinen Formel

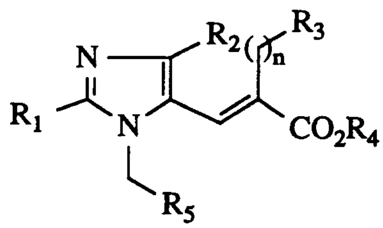 (I)

in welcher

- $R^1$ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder
  für Cycloalkyl mit 3 bis 8 Kohlenstoffatomen steht,
- $R^2$ für Wasserstoff, Halogen, Hydroxy, Nitro, Cyano, Trifluormethyl, Trifluormethoxy oder für Pentafluorethyl steht, oder
  für geradkettiges oder verzweigtes Alkyl mit bis zu 6 Kohlenstoffatomen steht, oder
  für Aryl mit 6 bis 10 Kohlenstoffatomen steht,
- $R^3$ für Cycloalkyl mit 3 bis 8 Kohlenstoffatomen oder
  für Heterocyclyl mit 3 bis 8 Kohlenstoffatomen und mit bis zu 3 Heteroatomen aus der Reihe S, O oder -N-A steht,
  worin
- A Wasserstoff oder geradkettiges oder verzweigtes Alkyl mit bis zu 6 Kohlenstoffatomen bedeutet,
- n für die Zahl 0, 1, 2, 3 oder 4 steht,
- $R^4$ für Wasserstoff, geradkettiges oder verzweigtes Alkyl mit bis zu 8 Kohlenstoffatomen oder Phenyl steht,
- $R^5$ für einen Rest der Formel

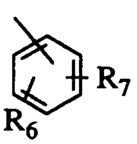 , 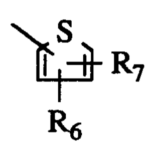 , 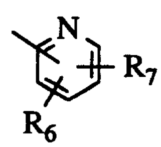 oder 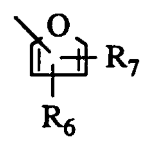

  steht, worin
- $R^6$ Wasserstoff, Halogen, Cyano, Nitro, Trifluormethyl, Hydroxy, Trifluormethoxy, für geradkettiges oder verzweigtes Alkyl oder Alkoxy mit jeweils bis zu 6 Kohlenstoffatomen bedeutet,
- $R^7$ Carboxy, geradkettiges oder verzweigtes Alkoxycarbonyl mit bis zu 6 Kohlenstoffatomen oder gegebenenfalls $C_1$-$C_4$-alkyliertes Tetrazolyl bedeutet,

und deren Salze.

**2.** Cycloalkyl- und Heterocyclyl substituierte Imidazolylpropensäurederivate nach Anspruch 1 wobei

- $R^1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, oder
  für Cyclopropyl, Cyclopentyl oder Cyclohexyl steht,
- $R^2$ für Wasserstoff, Fluor, Chlor, Brom, Iod, Trifluormethyl, Trifluormethoxy, Pentafluorethyl oder für geradkettiges oder verzweigtes Alkyl mit bis zu 6 Kohlenstoffatomen steht,
- $R^3$ für Cyclopropyl, Cyclopentyl, Cyclohexyl, Cycloheptyl, Dithiolanyl oder Pyranyl steht,
- n für die Zahl 0, 1, 2 oder 3 steht,
- $R^4$ für Wasserstoff oder für geradkettiges oder verzweigtes Alkyl mit bis zu 6 Kohlenstoffatomen steht,

$R^5$ für einen Rest der Formel

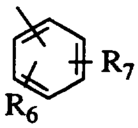 , 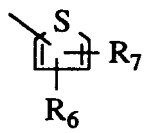 , 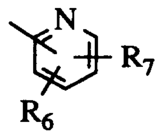 oder 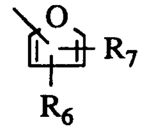

steht, worin

$R^6$ Wasserstoff, Fluor, Chlor, Brom, Trifluormethyl, Trifluormethoxy oder geradkettiges oder verzweigtes Alkyl mit bis zu 4 Kohlenstoffatomen bedeutet,

$R^7$ Carboxy, geradkettiges oder verzweigtes Alkoxycarbonyl mit bis zu 3 Kohlenstoffatomen oder Tetrazolyl bedeutet

und deren Salze.

**3.** Cycloalkyl- und Heterocyclyl substituierte Imidazolylpropensäurederivate nach Anspruch 1 wobei

$R^1$ für geradkettiges oder verzweigtes Alkyl oder Alkenyl mit jeweils bis zu 4 Kohlenstoffatomen oder Cyclopropyl steht,

$R^2$ für Wasserstoff, Fluor, Chlor, Brom, Iod, Trifluormethyl, Pentafluorethyl, Trifluormethoxy oder für geradkettiges oder verzweigtes Alkyl mit bis zu 4 Kohlenstoffatomen steht,

$R^3$ für Cyclopropyl, Cyclopentyl oder Cyclohexyl steht,

n für die Zahl 0, 1 oder 2 steht,

$R^4$ für Wasserstoff oder für geradkettiges oder verzweigtes Alkyl mit bis zu 4 Kohlenstoffatomen steht,

$R^5$ für einen Rest der Formel

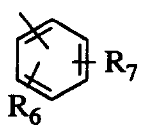 , 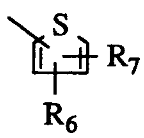 , 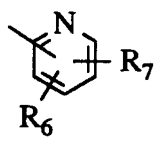 oder 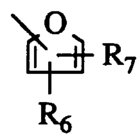

steht,
worin

$R^6$ Wasserstoff, Fluor, Chlor oder Brom bedeutet,

$R^7$ Carboxy, geradkettiges oder verzweigtes Alkoxycarbonyl mit bis zu 3 Kohlenstoffatomen oder Tetrazolyl bedeutet

und deren Salze.

**4.** Cycloalkyl- und Heterocyclyl substituierte Imidazolylpropensäurederivate nach Anspruch 1 zur therapeutischen Anwendung.

**5.** Verfahren zur Herstellung von Cycloalkyl- und Heterocyclyl substituierten Imidazolylpropensäurederivaten nach Anspruch 1, dadurch gekennzeichnet, daß man
Aldehyde der allgemeinen Formel (II)

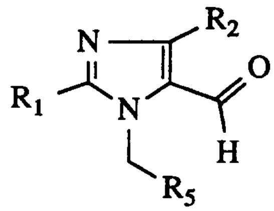

(II)

in welcher
$R^1$, $R^2$ und $R^5$ die in Anspruch 1 angegebene Bedeutung haben,
zunächst durch Umsetzung mit Verbindungen der allgemeinen Formel (III)

$R^3-(CH_2)_n-CO_2-T$ (III)

in welcher
$R^3$ und n die in Anspruch 1 angegebene Bedeutung haben,
und
T für geradkettiges oder verzweigtes Alkyl mit bis zu 8 Kohlenstoffatomen steht oder Phenyl bedeutet,
in inerten Lösemitteln, in Anwesenheit einer Base in die Verbindungen der allgemeinen Formel (IV)

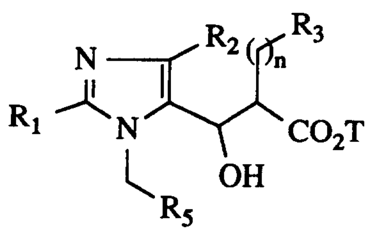

(IV)

in welcher
$R^1$, $R^2$, $R^3$, n und T die in Anspruch 1 angegebene Bedeutung haben,
überführt,
anschließend die freie Hydroxyfunktion durch Einführung einer Schutzgruppe blockiert und in einem letzten Schritt eine Eliminierung in inerten Lösemitteln in Anwesenheit einer Base durchführt,
und im Fall der Säuren ($R^4/R^7$ = H) die Ester verseift,
und gegebenenfalls die Substituenten $R^1$ und $R^2$ nach üblichen Methoden, beispielsweise durch Hydrierung oder Alkylierung variiert,
und im Fall, daß $R^7$ für alkyliertes Tetrazolyl steht, die -NH-Funktion alkyliert.

6. Verfahren nach Anspruch 5, dadurch gekennzeichnet, daß man in einem Temperaturbereich von -100 °C bis +100 °C arbeitet.

7. Arzneimittel enthaltend mindestens ein Cycloalkyl- oder Heterocyclyl substituiertes Imidazolylpropensäurederivat nach Anspruch 1.

8. Arzneimittel nach Anspruch 7 zur Behandlung von Hypertonie und Atherosklerose.

9. Verfahren zur Herstellung von Arzneimitteln nach Anspruch 7, dadurch gekennzeichnet, daß man die Cycloalkyl- oder Heterocyclcyl substituierten Imdidazolylpropensäurederivate gegebenenfalls mit Hilfe von üblichen Hilfs- und Trägerstoffen in eine geeignete Applikationsform überführt.

10. Verwendung der Cycloalkyl- und Heterocyclyl substituierten Imidazolylpropensäurederivate nach Anspruch 1 zur Herstellung von Arzneimitteln.